Europäisches Patentamt

European Patent Office

Office européen des brevets

(11) Publication number: **0 184 919**
A2

(12) **EUROPEAN PATENT APPLICATION**

(21) Application number: 85308686.6

(51) Int. Cl.⁴: **C 12 Q 1/26**

(22) Date of filing: 28.11.85

(30) Priority: 10.12.84 IL 73771

(43) Date of publication of application: **18.06.86**
**Bulletin 86/25**

(84) Designated Contracting States: **AT BE CH DE FR GB IT LI LU NL SE**

(71) Applicant: **YISSUM RESEARCH AND DEVELOPMENT COMPANY OF THE HEBREW UNIVERSITY OF JERUSALEM, 46, Jabotinsky Street P.O. box 4279, Jerusalem 91042 (IL)**

(72) Inventor: **Bachrach, Uriel, 33 Hatikva Street, Yemin Moshe Jerusalem (IL)**

(74) Representative: **Bizley, Richard Edward et al, BOULT, WADE & TENNANT 27 Furnival Street, London EC4A 1PQ (GB)**

(54) **Diagnostic kit for detecting amines and its production, and a method for detecting amines.**

(57) Methods and a kit are provided for the measurement of total polyamines, or of diamines and polyamines separately, in biological fluid samples. The methods are simple to carry out and extremely sensitive. The methods are applicable to the measurement of polyamines generally, and they may also be useful for the detection of neoplastic diseasse and for the monitoring of the results of therapeutic measures taken against such disease.

EP 0 184 919 A2

## DIAGNOSTIC KIT FOR DETECTING AMINES AND ITS

## PRODUCTION, AND A METHOD FOR DETECTING AMINES

The present invention relates to a method and kit for detecting amines. More particularly, the invention relates to a method and diagnostic kit for detecting the presence of diamines and polyamines in biological fluids after interfering materials have been removed. The method is simple and reliable and offers a degree of sensitivity heretofore unattainable. The method is applicable to the detection of polyamines for any purpose but is especially useful for the detection of neoplastic disease and for the monitoring of therapeutic measures designed to treat such disease.

The naturally occurring polyamines, i.e., putrescine, spermidine and spermine, and some of their derivatives, are present in all plant and animal tissues or cells. They are mainly associated with the nuclear material, and their levels increase in rapidly multiplying cells.

It is well known that cancerous tissues are rich in polyamines and that these compounds, or their conjugated derivatives, are excreted into the urine of cancer patients. The levels of polyamines (or of their derivatives) are also elevated in the blood of cancer patients and in the cerebrospinal fluid of patients suffering from brain tumors.

Recent studies have demonstrated that polyamines can be used for the assessment of the response to anti-tumor treatment. It has also been proposed that follow-up determinations may be useful in appraising relapse or recurrence of the disease prior to the appearance of clinical symptoms. In such studies, polyamine analysis may be employed for the early detection of cancer.

The quantitative determination of polyamines may also be of value in biological research, in monitoring growth or differentiation processes and in screening the activity of interferons, antiviral, antitumor or antiparasitic agents.

Several types of assays have been employed to determine polyamines. These include separation methods involving paper, gas or thin-layer chromatographic procedures as well

as electrophoretic and high-performance liquid chromatographic techniques. Recently, biological assays involving the oxidation of the polyamines by specific enzymes have come into favor, primarily because they are rapid and do not require sophisticated equipment. In these assays biological material containing polyamines is incubated with a specific enzyme, and the rate of oxidation is monitored by determining the concentration of the oxidation products. During the oxidation of the polyamines, aldehydes, ammonia and hydrogen peroxide are formed.

Such prior art methods are inefficient, however, since they require relatively laborious procedures for the extraction of the polyamines from biological material (or from hydrolysates of such material). Otherwise, interfering substances such as drugs etc. may inhibit the oxidation and produce false data. Moreover, most of the known assays are not very sensitive and require the use of relatively large amounts of biological material.

The present invention provides an assay method and a kit for the determination of polyamines which are applicable to routine use in clinical and research laboratories. This method is sensitive and simple and can be carried out with small sample volumes. Urine analysis, for example, may be performed with 0.1 to 0.2-ml samples. As little as 10 pmoles of polyamines per sample can be detected.

In addition to high sensitivity, the method of the invention provides a simple procedure for extracting polyamines from biological materials (or from hydrolysates of biological materials), thereby facilitating the removal of substances that could interfere with the assay.

In accordance with the present invention, a diagnostic test kit is provided for detecting the presence of diamines or polyamines in biological fluids, comprising:

a)   a negatively charged member adapted to absorb positively charged molecules of amines, diamines and polyamines;

b)   an eluting solution for removing amines, amino acids and derivatives thereof from the member;

c)   a strong electrolyte for eluting diamines and polyamines from the member;

d)   diamine and polyamine oxidizing enzymes for converting the amines to aldehydes, ammonia and hydrogen peroxide;

e)   a substance having peroxidative activity; and

f)   a substance undergoing luminescence in the presence of oxidized hydrogen peroxide to quantitatively determine the presence of diamines and/or polyamines which have been oxidized to hydrogen peroxide.

The present invention also provides a method for detecting the presence of diamines and polyamines in biological fluids, comprising:

a)   contacting a biological fluid sample with a negatively charged member adapted to absorb positively charged molecules of amines, diamines and polyamines;

b)   removing amines, amino acids and derivatives thereof from the member by rinsing with an amine-eluting solution;

c)   eluting diamines and polyamines from the member by contact with a strong electrolyte;

d)   oxidizing the eluted diamines and polyamines with oxidizing enzymes which convert the amines into aldehydes, ammonia and $H_2O_2$; and

e)   detecting $H_2O_2$ by reaction with a substance having peroxidative activity in the presence of a substance undergoing luminescence in the presence of oxidized $H_2O_2$, to quantitatively measure the diamines and/or polyamines which have been oxidized to produce hydrogen peroxide.

The present invention can also be used for detecting conjugated diamines or polyamines by simply heating the fluid containing the conjugated amines in the presence of an inorganic acid such as HCl to effect hydrolysis of the conjugated amines and to thereby release the amines for further processing according to steps a-e above.

Thus, this invention relates to a novel kit and a method for using the kit to quantify the levels of diamines and polyamines in biological fluid samples. Essentially, a negatively charged member is brought into contact with a biological fluid sample to bind all cationic materials in the sample such as amino acids, amines, diamines and polyamines. Then, the amino acids, amines and other compounds with a weak positive charge that could interfere with the assay are removed from the member by elution in a low ionic strength buffer. The more highly charged diamines and polyamines that remain bound to the member are then selectively eluted with a higher ionic strength buffer, and the eluate is treated with an oxidizing/peroxide-detecting system to quantify the diamines and polyamines released from the member.

The negatively charged member may consist of carboxymethyl cellulose or paper coated with polymers to which sulfonic functional groups have been attached, or other solid supports having other negatively charged functional groups. In a preferred embodiment, phosphocellulose sticks (Lev Scientific, Tel Aviv, Israel) are used.

Elimination of undesired and possibly interfering molecular species such as amines, amino acids and other compounds from the negatively charged member is carried out by elution in a strong electrolyte such as sodium chloride, magnesium chloride or ammonium hydroxide at low ionic strength. Typically, these electrolytes are used for this first elution step at a concentration of about 0.1 M. This step is preferably carried out at room temperature for about 30 minutes.

After the negatively charged members have been washed in the low ionic strength electrolyte, the concentration of the electrolyte may be increased stepwise to about 0.15 M, which elutes diamines, and then to about 1.0 M, which elutes polyamines. By such stepwise elution, the diamines and polyamines may be quantified separately. Alternatively, the higher ionic strength electrolyte may be used to elute both diamines and polyamines, for a total polyamine determination.

Once eluted both the diamines and the polyamines are quantified by an enzyme system consisting of either diamine or polyamine oxidase, and a substance having peroxidative activity. The latter substance is used in conjunction with a substance which luminesces in the presence of oxidized hydrogen peroxide.

While no particular source of the diamine and polyamine oxidizing enzymes is essential to the invention, diamine oxidase purified from pea seedlings, hog kidney, bacteria or fungi and polyamine oxidase from bovine plasma or serum, bacteria or fungi can be used. These enzymes convert diamines or polyamines to aldehydes, ammonia and hydrogen peroxide. In preferred embodiments, diamine oxidase is purified from pea seedlings, essentially as described by Hill [Methods in Enzymology, Volume 17B, Academic Press, New York, pp. 730-735 (1971)]. Polyamine oxidase is preferably purified from bovine plasma, essentially according to the method of Tabor et al. [Methods in Enzymology, Volume 2, Academic Press, New York, pp. 390-393 (1955)].

The substance having peroxidative activity may be an enzyme such as horseradish or fig latex peroxidase. Alternatively, urohemin, blood or molybdate ions may be used. The oxidized hydrogen peroxide produced by these substance is detected by a substance undergoing luminescence in the presence of oxidized hydrogen peroxide such as luminol (5-amino-2,3-dihydro-1,4-phthalazinedione). The luminescence of the compound can be detected by an instrument such as a Lumac Model 2010 Biocounter (Lumac B.V., 6370 AC Schaesberg, The Netherlands).

The method of the invention is applicable to both free and conjugated diamines and polyamines. Generally, biological fluid samples such as urine samples are first hydrolyzed by an inorganic acid such as hydrochloric acid, preferably at a final concentration of 1 N. This hydrolysis is preferably carried out for about 10 to 12 hours at a temperature of from about 100 to 110°C. If the hydrolysis step is omitted, the measured diamine and polyamine levels will be significantly lower, suggesting that part of the compounds are secreted as conjugates. These conjugates are probably acetyl derivatives. Once urine samples have been hydrolyzed, they can be kept at room temperature for several weeks without the loss of polyamines and diamines.

## EXAMPLES

The present invention may be more readily understood by reference to the following, non-limiting examples.

## EXAMPLE 1: PURIFICATION OF DIAMINE OXIDASE

The enzyme was purified from pea seedlings, essentially as described by Hill (see Section 4, above), as follows:

a)   Preparation of Crude Extract.

Pea seeds (approximately 1 kg) are soaked in running tap water for 18-24 hours and sown in rinsed sand at a depth of 4-6 mm.  The seeds are germinated in the dark. When the etiolated shoots are 2-5 mm tall (usually 10 days after sowing), the cotyledons and shoots (2-2.5 kg) are harvested, washed free of sand with cold tap water and minced in a Waring Blendor.  The homogenate is placed in a cotton cloth, and the juice is squeezed out.  The solid residue is mixed with 0.1 M potassium phosphate buffer, pH 7.0, and the liquid is squeezed out again.  The liquid extracts are pooled and frozen.

b)   Fractionation with Ethanol-chloroform Mixture The volume of crude extract is measured (generally 2000-3000 ml) and a 2:1 (v/v) ethanol:chloroform mixture (30 ml for each 100 ml of extract), cooled to -10°C, is added over a 30 minute period.  The mixture is allowed to stand in the cold for 1 hr, after which the inactive precipitate is removed by centrifugation at 3000 x g for 20 minutes.  The supernatant liquid is pooled, and ammonium sulphate (45 g/100 ml of supernatant liquid) is added at 10°C.  A solid separates and rises to the surface.  The lower layer is siphoned off, and the remaining slurry is centrifuged for 10 min at 3000xg.  The sediment is suspended in 0.02 M potassium phosphate buffer, pH 7.0 (1 ml/2 g. initial fresh pea seedlings), and kept at 2°C overnight.

c)   Precipitation with Ammonium Sulphate The suspension is stirred for 1 hour at 20°C, and the precipitate is removed by centrifugation for 20 minutes at 3000 x g.  The supernatant liquid is treated with ammonium sulphate (45 g/100 ml) and, after standing at 10°C for 1 hour, the precipitate is collected by centrifugation at 3000 x g for 20 minutes. The precipitate is suspended in about 20 ml of 0.2 M potassium phosphate buffer, pH 7.0, and then dialyzed first against cold running tap water for 3-4 hours and then against 3 changes (2 liters each) of 0.005 M potassium phosphate buffer, pH 7.0, over a period of 36 hours at 0-4°C.

        d)    Precipitation at pH 5.0.

The dialyzed material is centrifuged at 3000 x g, and the inactive sediment is discarded. The supernatant fluid is cooled to 5°C, adjusted to pH 5.0 by the slow addition of 0.05 N acetic acid, and allowed to stand for 1 hour at 4°C. The precipitate is collected by centrifugation, triturated with 10-15 ml of distilled water and brought to pH 7.0 by the dropwise addition of 0.05 N potassium hydroxide. The precipitation at pH 5.0 is repeated twice and the final solution is made up to a suitable volume (1 ml for each 100 g of pea seedlings) in 0.01 M potassium phosphate buffer, pH 7.0. This solution is finally freeze-dried and kept in vacuo in dark vials.

## EXAMPLE 2: PURIFICATION OF POLYAMINE OXIDASE

The enzyme was purified from bovine plasma essentially as described by Tabor et al.                  as follows:

        a)    Collection of Plasma.

Steer blood, obtained at the slaughterhouse, is treated immediately with 1/6 volume of citrate solution (8 g of citric acid and 26.7 g of Na citrate per liter of solution). The plasma is collected by centrifugation.

        b)    Fractionation with Ammonium Sulphate.

To 3,930 ml of plasma, 2,118 ml of saturated ammonium sulphate solution is added with stirring. After cooling to 3°C, the precipitate is removed by filtration through a fluted filter paper. To the filtrate, 3,760 ml of saturated ammonium sulphate is added. The precipitate is collected by filtration and dissolved in water (final volume 800 ml).

A second fractionation is carried out on this material, with the addition of the following volumes of saturated ammonium sulphate and the collection of each precipitate by filtration: (I) 185 ml, (II) 85 ml, (III) 75 ml, (IV) 100 ml and (V) 100 ml. Each precipitate is taken up in water and tested for activity. Precipitates (III) and (IV) are found to have the highest specific activities. They are pooled (total volume about 214 ml) and dialyzed for 48 hours against 3 changes of 0.01 M sodium acetate buffer (2.0 liters each).

c) Alcohol Precipitation

Portions (50 ml) of this dialyzed material are heated rapidly in a water bath to 65°C with stirring and maintained at this temperature for 10 minutes. The portions are then rapidly cooled to 0°C and combined. All alcohol precipitations are carried out 0°C with ethyl alcohol cooled at -10°C or below. After addition of 28.3 ml of 0.1 M $MnCl_2$, 283 ml is fractionated with ethanol. The following alcohol fractions are obtained by slow addition of the stated volumes of alcohol: (I) 142 ml of 25% alcohol; (II) 71 ml of 25% alcohol; (III) 71 ml of 25% alcohol and 14.2 ml of absolute alcohol and (V) 57 ml of absolute alcohol. The precipitates are collected by centrifugation and taken up in 30 ml of cold water. The fraction showing the highest specific activity (usually fraction IV) is then subjected to a second alcohol fractionation.

To this fraction (about 37.5 ml) is added 3.75 ml of 0.1 M $MnCl_2$. The following successive additions of cooled absolute alcohol are then made: (I) 1.91 ml; (II) 1.3 ml; (III) 1.13 ml; (IV) 1.63 ml; and (V) 1.63 ml. The resulting precipitates are collected by centrifugation and dissolved in 0.05 M phosphate buffer, pH 7.0.  Fractions containing the highest specific activities [fraction (III), (IV) and (V)] are pooled, freeze-dried and kept in vacuo in dark vials.

### EXAMPLE 3-MEASUREMENT OF TOTAL DIAMINES AND POLYAMINES

(a)  Hydrolysis

One ml of biological fluid, such as urine, is placed in a screw cap test tube along with 100 µl of concentrated hydrochloric acid.  The screw cap is tightened and the tube is heated in a thermoblock at 100-110°C overnight.

(b)  Application of Sample

Phospho-cellulose sticks (10 x 30 mm) are attached to a plastic holder, and 55 µl of the hydrolysates are applied onto each stick.  The sticks are dried at room temperature, by hot air, or in a vacuum oven.

(c)  Removal of Interfering Substances

Sodium borate buffer (0.02 M, pH 8.6) in 5000 ml quantities is poured into a plastic cylinder which is placed on a magnetic stirrer.  The holder containing the sticks with hydrolysates is immersed into the buffer, and the solution is stirred at room temperature for 30 minutes.  Rinsing is repeated twice with another batch of 5000 ml of sodium borate buffer.

The holder with the sticks is removed from the buffer and the sticks are gently blotted by pressing onto Whatman No. 1 filter paper.  Sticks are finally dried as in (b) above.

(d)  Elution of Diamines and Polyamines

The dry sticks are removed from the holder and each is transferred into a plastic tube containing 1.0 ml of 1 N sodium chloride in 0.2 M borate buffer, pH 8.6.

The tubes are incubated at 37°C with shaking for 30 minutes, the sticks are then removed from the fluid and drained, and excess buffer is removed by pressing each stick onto the walls of its test tube, using a glass rod or the tip of a plastic pipette.

(e) Luminescence

Aliquots of 25, 50 and 100 μl of the clear eluates are transferred into plastic luminescence measuring tubes. Volumes are made up to 100 μl by adding appropriate volumes (Table 1) of elution buffer (as in (d)).

Luminol ($10^{-4}$M) and peroxidase (30 μg/ml), 100 μl each, and 250 μl of $6.2 \times 10^{-2}$M glycine buffer, pH 8.6, are added to each tube, and the tubes are kept in the dark for at least 5 minutes until background luminescence fades.

For the assay of diamines (e.g., putrescine), diamine oxidase is used. Enzyme purified from pea seedlings (1-2 units/ml) is best, but diamine oxidase purified from hog kidney, bacteria, fungi or from other plants may also be used.

The tubes are transferred into the reading compartment of a Model 2010 Biocounter (Lumac B.V., 6370 AC Schaesberg, the Netherlands) or a similar luminometer, and background values are recorded at 30°C. The luminescence rate should be less than 300.

For the assay of diamines, 100 μl of diamine oxidase (1-2 units/ml) are injected into each tube. Integrated luminescence is immediately recorded for periods of 60 seconds each, until values drop to basal readings (less than 600 per 60 seconds).

0184919

-13-

Polyamines are similarly assayed except that polyamine oxidase is injected. An enzyme purified from bovine plasma (5-10 units/ml) is best, but polyamine oxidase purified from plants, bacteria, fungi or from other plants may similarly be employed.

f)    Standards and Internal Standards

A standard curve of putrescine, spermidine and spermine is prepared by spotting solutions ($10^{-4}$M or $10^{-5}$M) onto sticks and processing as above. If necessary, internal standards may be included in the assay by adding known amounts of standard polyamine solutions to the biological fluids prior to their application onto the stick.

g)    Calculation of results

Luminescence readings of the biological fluids are compared with those obtained with standards. The limit of detection is at least 10 pmoles per assay. For urine, results are expressed as nmoles/mg creatinine. Because spermidine constitutes most of the polyamines present in the urine, spermidine standards may be used for the calculation of urinary polyamines.

The composition of the assay mixture is shown in Table 1.

## TABLE 1

### COMPOSITION OF ASSAY MIXTURE

| Solution | Diamines Solution Volume (µl) | | | | Polyamines Solution Volume (µl) | | | |
|---|---|---|---|---|---|---|---|---|
| Eluate | 25 | 50 | 100 | - | 25 | 50 | 100 | - |
| Elution buffer | 75 | 50 | - | 100* | 75 | 50 | - | 100* |
| Glycine buffer | 250 | 250 | 250 | 250 | 250 | 250 | 250 | 250 |
| Luminol | 100 | 100 | 100 | 100 | 100 | 100 | 100 | 100 |
| Peroxidase | 100 | 100 | 100 | 100 | 100 | 100 | 100 | 100 |
| Injected diamine oxidase | 100 | 100 | 100 | 100 | | | | |
| Injected polyamine oxidase | | | | | 100 | 100 | 100 | 100 |

* This is the blank--one tube is enough for the entire set of sticks. Prior to the addition of diamine or polyamine oxidase, all samples are kept in the dark for at least 5 minutes.

EXAMPLE 4: QUANTIFICATION OF TOTAL POLYAMINES IN CANCER PATIENTS

Following the procedure of Example 3, urine samples from cancer patients were periodically tested after the onset of treatment. The normal level of total urinary polyamines is 25.1± 9.7 nmoles/mg creatinine. The results obtained are shown below in Table 2.

## TABLE 2

### POLYAMINE LEVELS IN CANCER PATIENTS

| Patient | Patient Status | Date | Total Polyamines (nmoles/mg creatinine) |
|---|---|---|---|
| Patient M.E. (Rectum cancer) | No progress after treatment | 8/1/84 | 64 |
| | | 8/5/84 | 148 |
| | | 8/9/84 | 160 |
| | | 8/13/84 | 285 |
| Patient C.I. (Glioblastoma) | No progress after treatment | 8/1/84 | 165 |
| | | 8/5/84 | 122 |
| Patient A.A. (deceased) (Lung cancer) | No progress after treatment | 7/30/84 | 308 |
| | | 8/6/84 | 218 |
| Patient P.E. (deceased) | No progress after treatment | 9/8/84 | 237 |
| | | 8/13/84 | 460 |
| Patient S.E. (sarcoma) | Improved condition | 7/20/84 | 18 |
| | | 7/25/84 | 16 |
| | | 8/1/84 | 29 |
| Patient Z.S. (Stomach cancer) | Stable condition | 8/1/84 | 60 |
| | | 8/8/84 | 56 |
| | | 8/13/84 | 57 |

EXAMPLE 5: SUCCESSIVE ELUTION OF DIAMINES AND POLYAMINES

a)      Hydrolysis and Application of Sample

The procedure is essentially that outlined in Example .3 (a) and (b) above.

b)      Removal of Interfering Substances

Ammonium hydroxide solution (0.1 M) in 2000-ml quantities is poured into a plastic cylinder which is placed on a magnetic stirrer in a hood.  The holder containing the sticks with the hydrolysates is immersed into the solution and stirred at room temperature for 30 minutes.  Then, the holder with the sticks is removed from the solution and the sticks are dried, first at room temperature and then with hot air in a vacuum oven.

c)      Elution of Diamines

Each dry stick is removed from the holder and placed in a plastic test tube containing 1.0 ml of 0.15 M magnesium chloride solution.  The tubes are incubated with shaking at 30°C minutes, and the sticks are removed from the fluid and dried as in step (b) above.

d)      Elution of Polyamines

The dry sticks are placed in a plastic tube containing 1.0 ml of 1.0 M magnesium chloride solution.  The tubes are incubated with shaking at 30°C for 30 minutes, and the sticks are removed and dried.

e)      Luminescence

Diamines are assayed in 0.15 M magnesium chloride solution, using diamine oxidase as described in Section 5.3 (e) above.  Enzyme purified from pea seedlings is best, but other diamine oxidase sources may be employed.

Polyamines are assayed in 1.0 M magnesium chloride solution using purified polyamine oxidase.  The enzyme purified from bovine plasma is best, but other polyamine oxidase sources may be employed.

The remainder of the assay is carried out essentially as described in  Example .3.

### EXAMPLE 6:  ASSAY OF FREE POLYAMINES

To determine levels of free diamines or polyamines, the hydrolysis step may be omitted.  Any biological fluid or cellular extract may be assayed, and samples may be concentrated or even dried using a Savant Evaporator.  For the assay of free diamines of polyamines in urine, the following procedure may be employed.

Aliquots of 200 µl of urine not hydrolyzed by acid are applied to sticks, and the sticks are dried as described above. Elution and luminescence measurements are then carried out as previously described.

### EXAMPLE 7:  ALTERNATIVE LUMINESCENCE PROCEDURE

When small amounts of enzyme are used or if samples contain compounds which slow down the oxidation of diamines or polyamines, an alternative approach for oxidation and luminescence readings may be used.

Samples are applied to sticks and eluted as described above, and the oxidation of diamines and polyamines is carried out as follows.

Eluates containing diamines or polyamines (50 to 100 µl volumes) are added to 100 µl-quantities of 0.2 M Tris-HCl buffer, pH 7.6, and diamine oxidase (for the assay of diamines) or polyamine oxidase (for the assay of polyamines) is added to the fluid in plastic test tubes.  Controls having eluates without enzyme and enzyme without eluate and standards are run in parallel, and all samples are incubated at 30°C for 60 minutes.

The tubes are then transferred into the measuring compartment of a luminometer, and the following solutions are injected into each tube:

| | |
|---|---|
| Sodium hydroxide (25 mM) | 100 µl |
| Peroxidase (1 mg/ml) | 100 µl |
| Luminol ($10^{-4}$M) | 100 µl |

Luminescence is recorded until basal background values are reached, and readings obtained with controls (enzyme without eluates and elutes without enzyme) are subtracted. Final values are compared with those obtained with standard solutions.

Many modifications and variations of the present invention may be made without departing from its spirit and scope, as will become apparent to those skilled in the art. The specific embodiments described herein are offered by way of example only, and the invention is limited only by the terms of the appended claims.

CLAIMS:

1. A diagnostic test kit for detecting the presence of diamines or polyamines in biological fluids, comprising:

(a) a negatively charged member adapted to absorb positively charged molecules of amines, diamines and polyamines;

(b) an eluting solution for removing amines, amino acids and derivatives therein from said member;

(c) a strong electrolyte for eluting diamines and polyamines from said member;

(d) diamine and polyamine oxidizing enzymes for converting said amines to aldehydes, ammonia and hydrogen peroxide;

(e) a substance having peroxidative activity; and

(f) a substance undergoing luminescence in the presence of oxidized hydrogen peroxide to quantitatively determine the presence of diamines and/or polyamines which have been oxidized to hydrogen peroxide.

2. A diagnostic kit according to claim 1, wherein said negatively charged member is phosphocellulose, carboxymethyl cellulose or paper coated with polymers having sulphonic functional groups.

3. A diagnostic kit according to claim 2, wherein said negatively charged member is a phospho- cellulose stick.

4.    A diagnostic kit according to any one of claims 1 to 3, wherein said strong electrolyte is sodium chloride, magnesium chloride or ammonium hydroxide.

5.    A diagnostic kit according to any one of claims 1 to 4, wherein said diamine and polyamine oxidizing enzymes are selected from diamine oxidase purified from pea seedlings, hog kidney, bacteria or fungi and polyamine oxidase purified from bovine plasma or serum, bacteria and fungi.

6.A diagnostic kit according to any one of claims 1 to 5, wherein said substance having peroxidase activity is selected from horse radish peroxidase, fig latex peroxidase, urohemin, blood iron and molybdate ions.

7.    A diagnostic kit according to any one of claims 1 to 6, wherein said substance undergoing luminescence is luminol.

8.    A diagnostic kit according to any one of claims 1 to 7 further comprising an inorganic acid for the hydrolysis of conjugated diamines and polyamines.

9.    A method for detecting the presence of diamines and polyamines in biological fluids, comprising:

(a)    contacting a biological fluid sample containing conjugated and unconjugated diamines and polyamines with a negatively charged member adapted to absorb positively charged molecules of amines, diamines and polyamines;

(b) removing amines, amino acids and derivatives thereof from said member by rinsing with an amine-eluting solution;

(c) eluting diamines and polyamines from said member by contact with a strong electrolyte;

(d) oxidizing said eluted diamines and polyamines with oxidizing enzymes to convert said amines into aldehydes, ammonia and $H_2O_2$; and

(e) detecting the $H_2O_2$ produced by the oxidation of the diamines and polyamines by reaction with a substance having peroxidative activity in the presence of a substance undergoing luminescence in the presence of oxidized $H_2O_2$.

10. A method according to claim 9, further comprising heating the biological fluid sample containing said conjugated diamines and polyamines in the presence of an inorganic acid to release diamines and polyamines prior to the carrying out of step a.

11. A method according to claim 10, wherein said acid is 1 N HCl.

12. A method according to claim 10 or claim 11, wherein said hydrolysis is carried out for about 10 to 12 hours at about $100^\circ$ to $110^\circ C$.

13. A method of producing a diagnostic kit for detecting the presence of diamines or polyamines in biological fluids, which method comprises providing in kit form:

(a) a negatively charged member adapted to absorb positively charged molecules of amines, diamines and polyamines;

(b) an eluting solution for removing amines, amino acids and derivatives therein from said member;

(c) a strong electrolyte for eluting diamines and polyamines from said member;

(d) diamine and polyamine oxidizing enzymes for converting said amines to aldehydes, ammonia and hydrogen peroxide;

(e) a substance having peroxidative activity; and

(f) a substance undergoing luminescence in the presence of oxidized hydrogen peroxide to quantitatively determine the presence of diamines and/or polyamines which have been oxidized to hydrogen peroxide.

14. A method according to claim 13 and further defined by the specific feature(s) of any one or more of claims 2 to 8.